Europäisches Patentamt

European Patent Office

Office européen des brevets

(11) Publication number: **0 220 962**
**A1**

## (12) EUROPEAN PATENT APPLICATION

(21) Application number: **86308383.8**

(22) Date of filing: **28.10.86**

(51) Int. Cl.4: **A 61 F 5/453**

(30) Priority: **28.10.85 GB 8526532**

(43) Date of publication of application:
**06.05.87 Bulletin 87/19**

(84) Designated Contracting States:
**CH DE ES FR IT LI SE**

(71) Applicant: **Negretti Aviation Limited**
**Edinburgh Place Edinburgh Way**
**Harlow Essex CM20 2DJ (GB)**

(72) Inventor: **Tweedie, John**
**300 Wharf Road**
**Ealand Crowle Scunthorpe, DN17 4JN (GB)**

**Mann, Ronald William**
**32 Greenhills**
**Harlow Essex, CM20 3SX (GB)**

(74) Representative: **Sommerville, John Henry**
**SOMMERVILLE & RUSHTON 11 Holywell Hill**
**St. Albans Hertfordshire, AL1 1EZ (GB)**

(54) **Urination facility.**

(57) The facility is of the kind in which a sheath 5 is worn on the penis and defines an aperture at its head end for connection to a collection container via a conduit.

There is a problem of leakage of urine past the base end of the sheath due to relative movement between the penis and sheath.

In accordance with this invention a membrane 1 generally in the shape of the frustum of a cone is provided which fits over the penis within the sheath and is of a length sufficient to accommodate said relative movement.

Preferably, the membrane is convoluted at 4, or otherwise shaped, along its length to facilitate relative movement.

The membrane can either be separate from the sheath, or integrally formed therewith at said base ends.

FIG.1

## Description

## URINATION FACILITY

This invention relates to urination facilities and in particular to a facility of the kind in which a sheath is worn on the penis which defines an aperture at its head end for connection to a bag, or other collection container, via a conduit to collect urine.

A sheath worn on the penis for the purpose of collecting and directing discharged urine requires a seal to prevent urine escaping between the base of the penis and the sheath.

Some arrangements are known in which the inner base end of the sheath is supported on an annular ring supported, or strapped, in position on the body of the user. The annular ring is provided with an internally directed relatively thick lip to engage around the penis. In such a case, even through relative movement between the base end of the penis and sheath is limited it has been found that an adequate seal was not possible. In an alternative arrangement, the inner end of the sheath is not supported on an annular ring, but is secured onto double-side adhesive tape, in turn secured around the penis. Although effective, it can be troublesome to fit and uncomfortable for the user, particularly upon removal.

An object of this invention is to provide a seal arrangement for the sheath which permits the penis to move in relation to the sheath whilst retaining the effectiveness of the seal.

According to this invention, a seal arrangement for the sheath of a urination facility of the kind described above comprises a tapered tubular membrane, generally in the shape of the frustum of a cone, for fitting over the penis within the sheath, the larger (base) end of said membrane being adapted to be supported close to the base of the penis and the other (head) end being sized to be an elastic fit on the penis, the length of the membrane being sufficient for relative movement between said base and head ends of the membrane to be accommodated.

The membrane may be separate from the sheath or be integrally formed therewith.

Preferably, to facilitate relative movement between its base and head ends, the membrane is convoluted or otherwise shaped along its length.

In order that the invention may be readily undestood, three embodiments thereof will now be described with reference to the accompanying drawing in which :-

     Figure 1 is a longitudinal elevation of one embodiment,

     Figure 2 is a longitudinal elevation of a second embodiment, and,

     Figure 3 is a longitudinal elevation of the third embodiment.

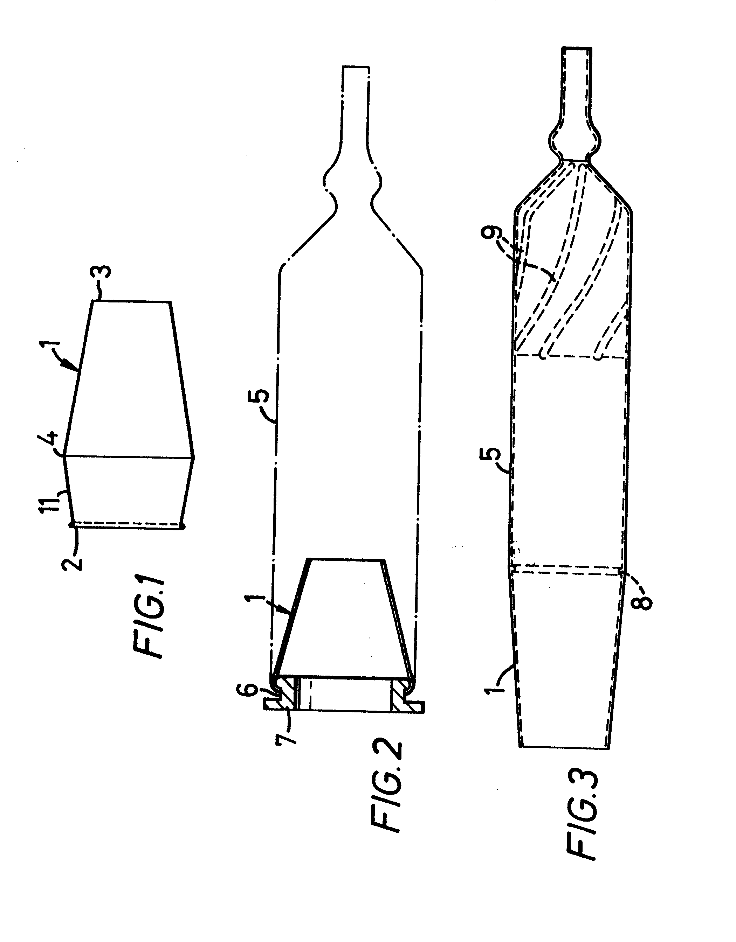

Referring to Figure 1 the membrane seal is formed as a dip moulding 1 of rubber latex or other appropriate elastic material. It is generally in the form of a thin wall, approximately 0.10 to 0.13mm, of frusto-conical shape and of a length sufficient to permit a range of relative movement between the base end 2 and head end 3. In this embodiment, the membrane has a convolution 4 intermediate its ends formed by part of its length, towards its base end, having a reverse taper 11. In this embodiment the membrane is separate from the sheath and its base end 2 is formed by a thickened bead of material which is adapted to be retained on a support ring as described below.

Referring to Figure 2, the membrane 1 is formed in a frusto-conical shape with no convolutions. It is also formed separately from the sheath, which is shown dotted at 5. As shown in the Figure both the seal 1 and the sheath 5 are retained in an annular groove 6 in a support ring 7 which latter, as discussed in our co-pending UK Patent Application 8626532, can be appropriately positioned in and supported by an undergarment such as an athletic support, or trunks.

Referring to Figure 3, the membrane seal 1 is formed as an integral part of a sheath 5. It will be seen that it is moulded axially with respect to the sheath and is defined by a moulded bead 8. After moulding the membrane is folded inwardly into the sheath so that the bead 8 forms the base end of the integrated sheath and membrane and is adapted to fit on a support ring as discussed above with reference to Figure 2. It will be seen that, in this embodiment, the sheath 5 is formed with spiralling internal ribs 9 to prevent occlusion in use. This feature is described and claimed in our said co-pending UK Patent Application No.8626532.

Due to the relative thinness and elasticity of the membrane discomfort is not experienced by the user and an effective seal is maintained and, furthermore, due to the length of the membrane, relative movement between its base and head ends can take place, thereby allowing relative movement of the penis to the base end support for the seal and sheath without affecting the seal.

## Claims

1. A seal arrangement for the sheath of a urination facility of the kind in which a sheath is worn on the penis which defines an aperture at its head-end for connection to a container, via a conduit, to collect urine, wherein the improvement comprises a tapered tubular membrane generally in the shape of the frustum of a cone for fitting over the penis within the sheath, the larger (base) end of said membrane being adapted to be supported close to the base of the penis and the other (head) end being sized to be an elastic fit on the penis, the length of the membrane being sufficient for relative movement between said base and head ends of the membrane to be accommodated.

2. A seal arrangement as claimed in claim 1, wherein the membrane is shaped along its length for facilitating relative movement between its said base and head ends.

3. A seal arrangement as claimed in claim 2, wherein the membrane is convoluted, which is formed by part of its length, towards its said base end, having a reverse taper.

4. A seal arrangement as claimed in claim 1, wherein said base end of the membrane is integrally formed with the base end of the sheath.

5. A seal arrangement as claimed in claim 1, wherein said membrane is separate from said sheath and an annular support ring is provided, adapted to be supported on the body around the base of the penis, said support ring defining a groove to which said base end of the membrane and the base end of the sheath can be elastically fitted.

FIG.1

FIG.2

FIG.3

0220962

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 083 122  (STILLE-WERNER)<br>* Whole document * | 1,2,4 | A 61 F    5/453 |
| Y | | 3,5 | |
| | --- | | |
| Y | FR-A-  912 396  (ROUSSEAU)<br>* Figure 2; page 2, lines 5-16 * | 3 | |
| | --- | | |
| Y | US-A-3 526 227  (APPELBAUM)<br>* Figures; claim 1 * | 5 | |
| | --- | | |
| A | GB-A-  871 862  (JOYNER) | | |
| | --- | | |
| A | FR-A-1 365 704  (WALLENBERG) | | **TECHNICAL FIELDS SEARCHED (Int. Cl.4)** |
| | --- | | |
| A | GB-A-  165 707  (MÜLLER) | | A 61 F |
| | ----- | | |

The present search report has been drawn up for all claims

| Place of search | Date of completion of the search | Examiner |
|---|---|---|
| THE HAGUE | 29-01-1987 | STEENBAKKER J. |